# EUROPÄISCHE PATENTSCHRIFT

(11) **EP 0 669 826 B1**
(45) Veröffentlichungstag und Bekanntmachung des Hinweises auf die Patenterteilung: **19.08.1998**
(21) Anmeldenummer: 93910008.7
(22) Anmeldetag: 17.05.1993
(51) Int. Cl.: A61K 31/66

(54) **(-)-METRIFONAT ENTHALTENDES ARZNEIMITTEL**
DRUG CONTAINING (-)-METRIPHONATE
MEDICAMENT CONTENANT DU (-)-METRIFONATE

(30) Priorität: 26.05.1992 DE 4217396
(43) Veröffentlichungstag der Anmeldung: 06.09.1995
(73) Patentinhaber: BAYER AG, 51368 Leverkusen (DE)
(72) Erfinder: BOBERG, Michael, D-5600 Wuppertal 1 (DE); KANHAI, Wolfgang, D-42115 Wuppertal (DE); KERN, Armin, D-5600 Wuppertal 1 (DE); MUSCHALEK, Volker, Dr., 42329 Wuppertal (DE); PLEISS, Ulrich, D-5603 Wülfrath (DE)
(86) Internationale Anmeldenummer: EP9301228
(87) Internationale Veröffentlichungsnummer: WO9324130

(56) Entgegenhaltungen:
- C.R. HEBD. SEANCES ACAD. SCI., SERIE C Bd. 280, Nr. 5, 1975, Seiten 291 - 292 J. BRIENNE ET AL 'Dédoublement du trichlorfon.' in der Anmeldung erw hnt
- KEIO J. MED. Bd. 36, Nr. 4, Oktober 1987, Seiten 381 - 391 E. GIACOBINI 'Modulation of brain acetylcholine levels with cholinesterase inhibitors as a treatment of alzheimer disease.'
- DRUG DEV. RES. Bd. 12, Nr. 3/4, 1988, Seiten 163 - 195 R.E. BECKER ET AL 'Mechanisms of cholinesterase inhibition in senile dementia of the Alzheimer type.'
- CLIN. PHARMACOKINET. Bd. 15, Nr. 2, 1988, Seiten 67 - 93 G. EDWARDS ET AL 'Clinical pharmacokinetics of anthelmintic drugs.'

## Beschreibung

Die Erfindung betrifft Arzneimittel, die als alleinigen Wirkstoff das (-)-enantiomere Metrifonat enthalten, deren Herstellung sowie deren Verwendung.

O,O-Dimethyl-2-trichlor-1-hydroxyethyl-phosphonat der Formel 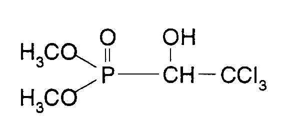 auch bekannt unter dem Namen Metrifonat, ist ein seit langer Zeit bekanntes Insektizid.

Darüber hinaus ist bekannt, daß Metrifonat anthelmintische Eigenschaften hat und deshalb zur Behandlung von durch bestimmte Wurmarten verursachte Erkrankungen (wie beispielsweise Bilharziose) sowohl in der Veterinär- als auch Humanmedizin verwendet werden kann.

In der US-Patentschrift US 4 950 658 wurde beschrieben, daß sowohl Metrifonat selbst als auch das durch Chlorwasserstoffabspaltung und Umlagerung von Metrifonat erhältliche 2,2-Dichlorvinyl-dimethylphosphonat der Formel 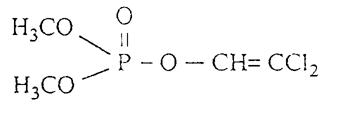 (auch als Dichlorfon oder DDVP bekannt) aufgrund ihrer Eigenschaft die Cholinesterase zu inhibieren zur Behandlung von seniler Demenz insbesondere vom Typ Morbus Alzheimer geeignet ist.

Außerdem ist bekannt, daß bei Wirkstoffen, die in verschiedenen Enantiomeren vorkommen können oft nur eines der Enantiomeren der Träger der eigentlichen Wirkung ist und das aus diesem Grund auch Registrierungsbehörden zunehmend die Entwicklung des wirksamen Enantiomeren und nicht des zumindestens 50% durch die unwirksame Komponente verunreinigten Racemates fordern (Schweiz. Med. Wochenschr., 120(5), 3.2.1990, 131-134; Pharm. Weekbl., 125(22), 1.6.1990, 552-554; Eur. J. Clin. Pharmacol., 41(2), 1991, 89-93).

Metrifonat besitzt ein asymmetrisches Kohlenstoffatom und kann somit in zwei Enantiomeren existieren. Sowohl bei der Anwendung als Insektizid als auch bei der therapeutischen Anwendung wird Metrifonat nur als Racemat eingesetzt. Wie für viele Wirkstoffe und hier insbesondere für Arzneimittel-Wirkstoffe bekannt ist, ist die eine enantiomere Form häufig wirksamer als die andere enantiomere Form, und in Einzelfällen ist die biologische Aktivität überhaupt nur einem der Enantiomeren zuzuschreiben.

Über die Enantiomeren des Metrifonat ist bisher nur sehr wenig bekannt. Utschik beschreibt die Existenz von Enantiomeren des Metrifonat im Zusammenhang mit Kristallmodifikationen, jedoch ohne die Enantiomeren selbst zu charakterisieren [H. Utschik Z.Chem. 23 (8), 207 (1983) und Z.Chem. 29 (1), 26(1989)].

1975 wurden die Enantiomeren erstmals gezielt synthetisiert und auf ihre biologische Aktivität bezüglich ihrer Cholinesterase-inhibierenden Eigenschaften im Vergleich zum Racemat untersucht. Die Autoren fanden, daß sich die Enantiomeren nur unwesentlich voneinander unterscheiden [J. Brieme et al. C.R. Hebd. Séances Acad. Sci., Sér. C, 280(5), 1975, 291-292].

Aus diesem Grund wurde bisher kein Vorteil darin gesehen, anstelle des bisher verwendeten Racemats eines der Enantiomeren von Metrifonat alleine anzuwenden.

Überraschenderweise wurde nun gefunden, daß entgegen der etablierten Meinung, wonach es kein Vorteil ist ein Metrifonat-Enantiomer alleine zu verwenden, die (-)-Form des Metrifonat ein wesentlich größeres pharmakologisches Potential besitzt, als zu vermuten war.

Es wurde gefunden, daß (-)-Metrifonat eine wesentlich höhere Stabilität gegenüber Wirkstoff-abbauenden (metabolisierenden) Enzymen besitzt als das (+)-Enantiomer. Beispielsweise konnte gezeigt werden, daß nach Umsetzung mit Lebermikrosomen des Menschen bis zu 20-mal mehr (-)-Metrifonat zur Verfügung steht als (+)-Metrifonat. Somit kann bei alleiniger Verwendung des (-)-Metrifonat als Wirkstoff eine wensentlich bessere Cholinesteraseihibition erreicht werden als bei Verwendung des Racemats bzw. des (+) Enantiomeren.

Damit kann man bei Verwendung des (-)-Metrifonat anstelle des Racemats zur Erzielung der gleichen Wirkung mit wesentlich weniger, sogar weniger als der Hälfte Wirkstoff auskommen und die Patienten werden wesentlich weniger belastet, insbesondere bei Dauerbelastung.

Darüber hinaus ist auch von Vorteil, daß Arzneimittel, die nur (-)-Metrifonat enthalten, ein geringeres Volumen als die Racemat-haltigen haben, was sich beispielsweise positiv auf die Tablettengröße auswirkt.

Die Metrifonat-Enantiomeren können entweder nach der in der Literatur angegebenen Methode synthetisiert werden [J. Brieme et al. C.R. Hebd. Séances Acad. Sci., Sér.C 280(5), 291(1975)], oder aus dem Racemat durch Trennung an einem chiralen Adsorbens gewonnen werden.

Die Erfindung betrifft (-)-Metrifonat-haltige Arzneimittel, wobei hierunter pharmazeutische Zubereitungen zur oralen, parenteralen oder transdermalen Applikation zu verstehen sind, die neben dem Wirkstoff (-)-Metrifonat geeignete Hilfs- und Trägerstoffe enthalten. Als Beispiele seien hier zu nennen: Tabletten, Dragees, Pillen oder Kapseln, gegebenenfalls jeweils als Retard-Form. Suppositorien, Pflaster oder Injektionslösungen.

Der Wirkstoff (-)-Metrifonat soll in diesen Zubereitungen in einer Konzentration von 0,1 bis 99,5 Gew.-%, bevorzugt von 0,5 bis 95 Gew.-% der Gesamtmischung vorhanden sein.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den Wirkstoff (-)-Metrifonat in Gesamtmengen von etwa 0,01 bis etwa 10 mg/kg, bevorzugt in Gesamtmengen von etwa 0,1 mg/kg bis 5 mg/kg. besonders bevorzugt von 0,5 bis 2,5 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ebenso ist es möglich, Retard-Formulierungen zur oralen Applikation herzustellen oder transdermale Applikationssysteme wie beispielsweise Pflaster zu verwenden.

### Beispiel 1 / Tabletten

| | |
|---|---|
| Metrifonat | 10,0 mg |
| Mikrokristalline Cellulose | 34,0 mg |
| Calciumphosphat | 20,0 mg |
| Natriumphosphat | 3,0 mg |
| Na-Carboxy-Methylcellulose | 3,0 mg |

### Beispiel 2 / Kapseln

| | |
|---|---|
| Metrifonat | 10,0 mg |
| Avicel | 24,0 mg |
| Laktose | 55,0 mg |
| Maisstärke | 10,0 mg |
| Aerosil | 0,5 mg |
| Mg-Stearat | 0,5 mg |
| Hartgelatinekapsel Gr. 5 | |

## Patentansprüche

1. Metrifonat enthaltendes Arzneimittel, dadurch gekennzeichnet, daß es Metrifonat nur in der (-)-Form neben üblichen Träger- und Hilfsstoffen enthält.

2. Arzneimittel nach Anspruch 1 oder 2, dadurch gekennzeichnet, daß es in Form einer für die orale Applikation geeignete Zubereitung vorliegt.

3. Arzneimittel nach Anspruch 1 oder 2, dadurch gekennzeichnet, daß es in Form einer für eine transdermale Applikation geeigneten Zubereitung vorliegt.

4. Arzneimittel nach Anspruch 1 oder 2, dadurch gekennzeichnet, daß es in Retardform vorliegt.

5. Arzneimittel nach Anspruch 1 oder 2, dadurch gekennzeichnet, daß es in einer injizierbaren Form vorliegt.

6. Verwendung von (-)-Metrifonat als alleiniger Wirkstoff zur Herstellung eines Arzneimittels.

7. Verwendung von (-)-Metrifonat als alleinigen Wirkstoff zur Herstellung von Arzneimitteln zur Behandlung der Alzheimerschen Krankheit.

8. Verfahren zur Herstellung von Arzneimitteln nach Anspruch 1, dadurch gekennzeichnet, daß man (-)-Metrifonat mit den üblichen Hilfs- und Trägerstoffen in eine geeignete Applikationsform überführt.

## Claims

1. Metrifonate-containing medicament, characterized in that, besides customary auxiliaries and excipients, it contains metrifonate only in the (-)-form.

2. Medicament according to Claim 1, characterized in that it is present in the form of a preparation suitable for oral administration.

3. Medicament according to Claim 1, characterized in that it is present in the form of a preparation suitable for transdermal application.

4. Medicament according to Claim 1, characterized in that it is present in sustained-release form.

5. Medicament according to Claim 1, characterized in that it is present in an injectable form.

6. Use of (-)-metrifonate as the sole active compound for the production of a medicament.

7. Use of (-)-metrifonate as the sole active compound for the production of medicaments for the treatment of Alzheimer's disease.

8. Process for the production of medicaments according to Claim 1, characterized in that (-)-metrifonate is converted into a suitable administration form with the customary auxiliaries and excipients.

## Revendications

1. Médicament contenant du métrifonate, caractérisé en ce qu'il contient du métrifonate uniquement sous la forme (-), à côté de supports et de substances auxiliaires classiques.

2. Médicament suivant la revendication 1 ou 2, caractérisé en ce qu'il se présente sous la forme d'une préparation qui convient pour l'administration orale.

3. Médicament suivant la revendication 1 ou 2, caractérisé en ce qu'il se présente sous la forme d'une préparation qui convient pour l'administration transdermique.

4. Médicament suivant la revendication 1 ou 2, caractérisé en ce qu'il se présente sous une forme à action retardée.

5. Médicament suivant la revendication 1 ou 2, caractérisé en ce qu'il se présente sous une forme injectable.

6. Utilisation du (-)-métrifonate comme unique substance active pour la préparation d'un médicament.

7. Utilisation du (-)-métrifonate comme unique substance active pour la préparation de médicaments destinés au traitement de la maladie d'Alzheimer.

8. Procédé de préparation de médicaments suivant la revendication 1, caractérisé en ce qu'on fait prendre une forme administrable appropriée à du (-)-métrifonate, avec les substances auxiliaires et les supports classiques.
